# DEMANDE DE BREVET EUROPEEN

(11) **EP 4 147 646 A1**
(43) Date de publication de la demande: **15.03.2023**
(21) Numéro de dépôt: 22194239.4
(22) Date de dépôt: 06.09.2022
(51) Int. Cl.: A61B 8/00

(54) **DISPOSITIF PIVOTANT POUR UNE SONDE D ÉCHOGRAPHIE**

(30) Priorité: 09.09.2021 FR 2109429
(71) Demandeur: SUPERSONIC IMAGINE, 13290 Aix-en-Provence (FR)
(72) Inventeur: LE GARREC, Morgane, 13090 Aix-en-Provence (FR); MAJOREL, Damien, 30100 Ales (FR); MARTIN, Jean-Alain, 84120 Pertuis (FR)
(74) Mandataire: Paustian & Partner Patentanwälte mbB

(57) **Abrégé**

La présente divulgation concerne un dispositif pour une sonde d'échographie pour acquérir des données d'échographie d'un milieu, dans lequel le dispositif est configuré pour être associé à la sonde d'une manière pivotante, tel que l'angle entre la sonde et la surface du milieu peut être ajusté et/ou fixé en pivotant le dispositif.

## Description

### Domaine Technique

La présente invention est relative aux systèmes médicaux et aux procédés utilisant un tel système. Un tel système peut constituer un système d'examen médical. Plus particulièrement, il peut constituer un système d'échographie.

### Technique antérieure

Un système d'échographie comprend habituellement des moyens électroniques, par exemple un capteur et/ou une sonde d'échographie pour acquérir des données d'un patient, et un processeur pour traiter des données acquises.

Un système d'échographie peut être destiné à fournir des données d'un milieu à examiner. Des exemples de tels systèmes comprennent un système d'imagerie optique, un système d'imagerie par ultrasons, un système de radiographie, un système de tomographie par ordinateur, un système de mammographie et autres. Dans le cas des applications médicales, le milieu est un corps, par exemple une partie du corps d'un patient (muscles, fœtus, sein, foie, abdomen, ...).

En général, la sonde est maintenue contre la surface d'un milieu observé, afin d'acquérir des données sur ce milieu. Dans ce contexte, il est important de ne pas déplacer la sonde pendant l'acquisition des données, ou de la déplacer uniquement d'une manière spécifique et désirée.

Par exemple, dans le cas d'une imagerie ultrasonore classique en mode B (en anglais « Brightness mode »), il est souhaitable que la sonde reste dans une position fixe pendant l'acquisition des données.

Par ailleurs, des méthodes d'imagerie ultrasonore sont connues, dans lesquelles une déformation est appliquée au milieu pendant l'acquisition des données, par exemple décrit par WO2021116326A2. Dans cette méthode, l'élasticité non linéaire d'un milieu est quantifiée en utilisant des ondes de cisaillement (en anglais « non-linear shear wave elastography » ; NL-SWE). La méthode comprend les étapes suivantes :
A1. Collecte d'une succession temporelle de données d'élasticité par ondes de cisaillement du milieu, A2. Application d'une déformation successivement changeante au milieu selon une séquence de déformations prédéterminée pendant la collecte des ondes de cisaillement, A3. Observation de l'évolution de déformation réelle, et B. Quantification de l'élasticité non linéaire du milieu en fonction de la succession temporelle de données et l'évolution de déformation.

Aussi dans une mode NL-SWE, il peut être souhaitable que la sonde reste dans une position fixe pendant l'acquisition des données.

Cependant, il peut être difficile d'éviter tout mouvement indésirable de la sonde pendant l'acquisition des données, en particulier pendant une étape de déformation. Par exemple, le gel ultrasonore utilisé peut rendre la surface du milieu glissante. De même, une lésion dure dans le milieu ou la respiration du patient peut entraîner des mouvements indésirables. Ainsi, les mouvements indésirables peuvent notamment engendrer un mouvement transversal de la sonde (par exemple un glissement sur la surface du milieu) et/ou un mouvement de rotation modifiant l'angle d'inclinaison.

De plus, étant donné que lors de la déformation, la force est appliquée uniquement à la surface de la sonde, une homogénéité de la déformation est difficile à atteindre.

Par ailleurs, le lancement de l'acquisition de données nécessite souvent une action de l'utilisateur sur l'interface utilisateur (par exemple pour appuyer sur un bouton) synchronisée avec le début de la déformation.

Différents accessoires pour sondes à ultrasons sont connus. Par exemple, EP3202326A1 décrit un accessoire pour sonde à ultrasons. L'accessoire comprend un élément d'adaptation acoustique prévu sur un émetteur-récepteur d'une sonde à ultrasons et déformé sur la base d'une surface d'une cible d'inspection lorsque la sonde à ultrasons balaie la surface de la cible d'inspection.

En outre, le document EP1629777A1 divulgue un mécanisme de pression pour appliquer une pression à la surface de contact d'un sujet perpendiculairement à la surface d'émission/réception d'ondes ultrasonores d'une sonde à ultrasons via la surface d'émission/réception d'ondes ultrasonores.

Cependant, les accessoires connus ne sont pas adaptés pour surmonter les problèmes mentionnés ci-dessus, notamment pour appliquer une pression homogène sur la surface du milieu et en même temps maintenir la sonde dans un angle choisi par rapport à la surface du milieu.

### Exposé de l'invention

La présente divulgation a donc pour but de prévoir un dispositif pour une sonde d'échographie qui permet de maintenir la sonde dans un angle choisi par un utilisateur pendant l'acquisition des données, et qui permet une déformation homogène du milieu pendant l'acquisition de données en maintenant l'angle choisi.

La présente divulgation se rapporte à un dispositif (accessoire) pour une sonde d'échographie pour acquérir des données d'échographie d'un milieu, dans lequel le dispositif est configuré pour être associé à la sonde d'une manière pivotante, tel que l'angle entre la sonde et une surface du milieu peut être ajusté et/ou fixé en pivotant le dispositif.

Grâce à ce dispositif, un utilisateur peut stabiliser et maintenir la sonde dans un angle / inclinaison prédéfini(e) pendant l'acquisition de données.

Par exemple, dans le cas d'une imagerie ultrasonore classique en mode B (en anglais « Brightness mode »), il est souhaitable que la sonde reste dans une position fixe pendant l'acquisition des données. En fonction de de la localisation de la région d'intérêt du milieu qui doit être imagée, il peut également être avantageux que la sonde ne soit pas placée perpendiculairement à la surface du milieu mais avec un angle d'inclinaison choisi. Dans ce cas, le dispositif de la présente divulgation permet que l'angle d'inclinaison soit maintenu pendant l'acquisition des données.

Selon un autre exemple, dans le mode NL-SWE, il peut être souhaitable de placer la sonde avec un angle d'inclinaison sélectionné par rapport au milieu pour acquérir les données d'une région d'intérêt spécifique, une lésion par exemple. En conséquence, le dispositif de la présente divulgation permet de conserver l'angle d'inclinaison sélectionné, pendant la ou les étapes de déformation.

Notamment, le dispositif peut être configuré pour être associé à la sonde d'une manière pivotante, tel que, quand (ou pendant que) le dispositif est disposé contre (et/ou maintenu sur) la surface du milieu observé, l'angle entre la sonde et la surface du milieu observé peut être ajusté et/ou fixé en pivotant le dispositif.

La sonde d'échographie peut collaborer avec un système qui est configuré pour traiter des données d'un milieu acquises par la sonde.

Le dispositif peut être configuré pour être positionné et/ou attaché à la sonde d'une manière pivotante. Donc, « associé à la sonde d'une manière pivotante » peut signifier selon un exemple « positionné et/ou attaché à la sonde d'une manière pivotante ».

Le dispositif peut comprendre en outre des moyens adaptables configurés pour attacher le dispositif à des coques de sondes différentes.

La sonde peut être configurée pour émettre des ondes ultrasonores et/ou peut comprendre un transducteur configuré pour émettre des ondes ultrasonores sur un premier côté de la sonde.

Le dispositif peut être configuré pour être positionné et/ou attaché sur le premier côté de la sonde d'une manière pivotante. Donc, le dispositif peut être configuré pour être positionné et/ou attaché par exemple sur la coque de la sonde et permettant le pivotement de celle-ci.

Alternativement, le dispositif peut aussi être configuré pour être positionné et/ou attaché d'une manière pivotante sur un autre côté que le premier côté de la sonde. Cette configuration peut s'appliquer par exemple, si la sonde est configurée pour des zones endocavitaires. Par exemple, le dispositif peut être configuré pour être positionné et/ou attaché à la poignée de la sonde ou adjacente à la poignée. Alternativement, le dispositif peut aussi être configuré pour être positionné et/ou attaché d'une manière pivotante sur un côté opposé au premier côté.

L'angle peut être compris comme un angle d'inclinaison entre la direction des ondes ultrasonores émises par la sonde et la surface du milieu.

Le dispositif peut comprendre une ouverture configurée pour permettre la transmission des ondes ultrasonores. Donc, l'ouverture peut être configurée telle que l'émission et la réception des ondes ultrasonores soit possible sans perturbation.

Le dispositif peut comprendre un plateau et/ou une surface plane configuré(e) pour être disposé(e) contre la surface du milieu et pour stabiliser l'angle entre la sonde et la surface du milieu.

Dans par exemple des modes similaires au mode NL-SWE, le dispositif peut donc permettre une déformation homogène du milieu et, par conséquent, assurer une bonne qualité d'image. En effet, lors de la déformation avec une sonde sans le dispositif, seule la zone tissulaire située à l'aplomb de la sonde serait comprimée, ce qui peut perturber l'estimation de la déformation si le plan d'imagerie varie pendant l'acquisition.

Le dispositif et/ou son ouverture peut comprendre un moyen de couplage configuré pour permettre la transmission des ondes ultrasonores.

Le moyen de couplage peut comprendre un film et/ou un coussinet de gel, et optionnellement un adhésif pour attacher le film et/ou le coussinet de gel au dispositif.

Selon un exemple, le moyen de couplage peut comprendre un film bi-matière avec une ou plusieurs parties adhérentes positionnées dans une zone périphérique. Le film bi-matière peut comprendre en outre une partie configurée pour coupler le dispositif au milieu et/ou pour permettre la transmission des ondes ultrasonores. Cette dernière partie peut être positionnée dans une zone où des ondes ultrasonores sont émises par la sonde.

Le dispositif et/ou son ouverture peut comprendre un moyen de stabilisation configuré pour optimiser la stabilité du dispositif sur la surface du milieu.

Le moyen de stabilisation peut comprendre une partie adhérente sur une surface du dispositif configurée pour être disposée contre la surface du milieu.

Le moyen de stabilisation peut donc éviter que la sonde et le dispositif glissent sur la surface du milieu. Le dispositif peut donc assurer une acquisition de données sans mouvement indésirable de la sonde par rapport du milieu pendant l'acquisition, comme par exemple une rotation (empêchée par le plateau optionnel du dispositif) et un mouvement transversal (empêché par le moyen de stabilisation optionnel du dispositif).

Le dispositif peut être configuré pour ajuster et/ou fixer l'angle en utilisant des moyens mécaniques et/ou électromécaniques et/ou électroniques.

Les moyens mécaniques peuvent comprendre un embrayage pour le réglage de l'angle et/ou pour fixer l'angle.

Le dispositif peut être configuré pour un réglage de l'angle par étapes ou en continu, par exemple en contrôlant l'embrayage.

Le dispositif peut comprendre en outre un capteur de température configuré pour mesurer la température de la surface du milieu et/ou à la surface de la sonde.

Le dispositif peut comprendre en outre un capteur de pression configuré pour mesurer une pression appliquée à la surface du milieu par le dispositif.

Le dispositif peut comprendre en outre un indicateur de pression configuré pour indiquer un niveau de pression appliquée à la surface du milieu.

Le dispositif peut comprendre en outre une pluralité de capteurs de pression locale. Chaque capteur de pression locale peut être configuré pour mesurer une pression appliquée à la surface du milieu par une zone différente du dispositif. Le dispositif peut comprendre en outre optionnellement une pluralité d'indicateurs de pression locale, dont chaque indicateur de pression est configuré pour indiquer un niveau de pression locale.

L'indicateur de pression et/ou les indicateurs de pression locale et/ou un ou plusieurs indicateurs de température peuvent être disposés sur un côté opposé d'une surface libre du dispositif qui est disposée contre le milieu.

Le dispositif peut comprendre en outre un capteur d'inclinaison configuré pour mesurer l'angle entre la sonde et la surface du milieu.

Le dispositif peut comprendre en outre un afficheur pour indiquer cet angle.

Le dispositif peut comprendre en outre un système de guidage de positionnement configuré pour indiquer une position cible à l'utilisateur de la sonde vers la position cible.

Le système de guidage de positionnement peut comprendre des indicateurs de direction situés à la surface libre du dispositif et configurés pour indiquer la direction vers la position cible.

Le système de guidage de positionnement peut comprendre en outre des moyens de localisation configurés pour localiser la position actuelle du dispositif par rapport au milieu.

Le système de guidage de positionnement peut être configuré pour communiquer avec une unité de traitement externe pour transmettre des données de localisation et/ou pour recevoir des données d'une position cible et/ou d'une direction vers la position cible. Cette unité de traitement externe peut constituer par exemple un système qui collabore / communique avec la sonde d'échographie et qui est configuré pour traiter des données d'un milieu acquises par la sonde.

Le dispositif peut comprendre en outre un support ergonomique, par exemple un coussin, configuré tel qu'une main tenant la sonde puisse reposer dessus.

La présente divulgation se rapporte également à une sonde d'échographie pour acquérir des données d'échographie d'un milieu. La sonde peut être configurée pour collaborer et/ou communiquer avec un système destiné à traiter les données d'échographie. La sonde peut comprendre un dispositif selon la divulgation présente.

La présente divulgation se rapporte également à un système d'échographie, comprenant un dispositif selon la divulgation présente. Le système peut optionnellement comprendre une sonde d'échographie pour acquérir des données d'un milieu. En outre, le système peut comprendre des moyens de localisation configurés pour localiser la position du dispositif par rapport au milieu. Ce système peut constituer par exemple un système qui collabore et/ou communique avec la sonde d'échographie et qui est configuré pour traiter des données d'un milieu acquises par la sonde.

La présente divulgation se rapporte également à un procédé pour acquérir des données d'un milieu par une sonde d'imagerie en utilisant un dispositif associé à la sonde d'une manière pivotante, comprenant les étapes :
A. ajuster et/ou fixer l'angle entre la sonde et la surface du milieu en pivotant le dispositif,
B. acquérir des données du milieu en utilisant la sonde.

En conséquence, l'angle peut d'abord être ajusté et/ou fixé. Ensuite, des données peuvent être acquises par la sonde. Ainsi, il est possible d'acquérir des données à un angle d'inclinaison stable.

Les étapes A, et B peuvent être réitérées.

Le procédé peut comprendre en outre les étapes :
C1. changer un niveau de déformation du milieu appliqué par le dispositif, et/ou
C2. changer l'angle.

Les étapes C1 et/ou C2 peuvent être réalisées après l'étape B.

L'étape B peut être réitérée après l'étape C1. Par conséquent, en changeant le niveau de déformation selon l'étape C1, et en répétant l'étape B, les données peuvent être acquises à deux niveaux de déformation différents.

Dans le cas où les étapes B et C1 sont réitérées, les données peuvent être acquises à plus de deux niveaux de déformation différents. Une telle méthode peut être utile par exemple dans un mode NL-SWE.

L'étape B peut également être réitérée après l'étape C2. Par conséquent, en changeant l'angle d'inclinaison selon l'étape C2, et en répétant l'étape B, les données peuvent être acquises à deux angles d'inclinaison différents de la sonde.

Dans le cas où les étapes B et C2 sont réitérées, les données peuvent être acquises à plus de deux angles d'inclinaison différents. Une telle méthode peut être utile, par exemple, dans un mode de collecte d'images 3D qui est basé sur l'acquisition d'un ensemble de données d'images 2D d'un milieu à différents angles de la sonde par rapport au milieu.

En option, le procédé peut aussi comprendre une réitération des étapes A, B et C1 et/ou C2, dans laquelle, dans l'étape supplémentaire A, l'angle est ajusté et/ou fixé, avant que les données ne soient acquises dans l'étape B.

Cependant, un ajustement et/ou fixation de l'angle peut aussi être effectué déjà dans l'étape C2. Donc, l'étape C2 peut aussi être compris comme une réitération de l'étape A.

Les caractéristiques et avantages de l'invention apparaitront à la lecture de la description qui va suivre, donnée uniquement à titre d'exemple non limitatif, et faite en référence aux figures annexées. Notamment, les exemples illustrés dans les figures peuvent être combinés sauf incohérence notoire.

### Brève description des figures

[Fig. 1] montre schématiquement une sonde conventionnelle dans une première vue latérale.
[Fig. 2a] montre schématiquement une vue en perspective d'un premier mode de réalisation d'un dispositif selon la présente divulgation.
[Fig. 2b] montre schématiquement une première vue en coupe du dispositif de la figure 2a, dans lequel un mouvement de pivotement du dispositif est illustré.
[Fig. 3a] montre de manière schématique une première vue latérale d'un deuxième mode de réalisation d'un dispositif selon la présente divulgation, dans lequel le dispositif peut avoir un film de couplage.
[Fig. 3b] montre de manière schématique une première vue latérale d'un troisième mode de réalisation d'un dispositif selon la présente divulgation, dans lequel le dispositif peut avoir un coussinet de gel.
[Fig. 4a] montre schématiquement une deuxième vue latérale d'un quatrième mode de réalisation d'un dispositif selon la présente divulgation, dans lequel le dispositif peut avoir un moyen de stabilisation configuré pour optimiser la stabilité du dispositif sur la surface du milieu.
[Fig. 4b] représente schématiquement une vue de dessous du dispositif de la figure 4a.
[Fig. 5] montre schématiquement une première vue latérale d'un cinquième mode de réalisation d'un dispositif selon la présente divulgation, dans lequel le dispositif peut avoir un moyen mécanique pour un pivotement par étapes/paliers et/ou d'indexage.
[Fig. 6a] montre schématiquement une deuxième vue latérale d'un sixième mode de réalisation d'un dispositif selon la présente invention, dans lequel le dispositif peut être configuré pour un pivotement continu et verrouillable en n'importe quelle position
[Fig. 6b] montre schématiquement une première vue latérale du dispositif de la figure 6a avec un capteur d'inclinaison optionnel.
[Fig. 6c] montre schématiquement une vue de dessus du dispositif des figures 6a et 6b avec un indicateur d'inclinaison optionnel.
[Fig. 7a] montre schématiquement une première vue latérale d'un septième mode de réalisation d'un dispositif selon la présente divulgation, dans lequel le dispositif peut avoir des capteurs de pression et des indicateurs de pression.
[Fig. 7b] montre schématiquement une vue de dessus du dispositif de la figure 7a et en particulier la disposition des indicateurs de pression.
[Fig. 8a] montre schématiquement une première vue latérale d'un huitième mode de réalisation d'un dispositif selon la présente divulgation, dans lequel le dispositif peut avoir un capteur de positionnement et des indicateurs de direction.
[Fig. 8b] montre schématiquement une vue de dessus du dispositif de la figure 8a.
[Fig. 9] montre schématiquement une première vue latérale d'un neuvième mode de réalisation d'un dispositif selon la présente divulgation, dans lequel le dispositif peut avoir un support ergonomique permettant de reposer le poignet.

### Description des modes de réalisation

Sur les différentes figures, fournies à titre d'illustration, les mêmes références numériques désignent des éléments identiques ou similaires. Les différents modes de réalisation peuvent être combinés de n'importe quelle manière, sauf description contraire.

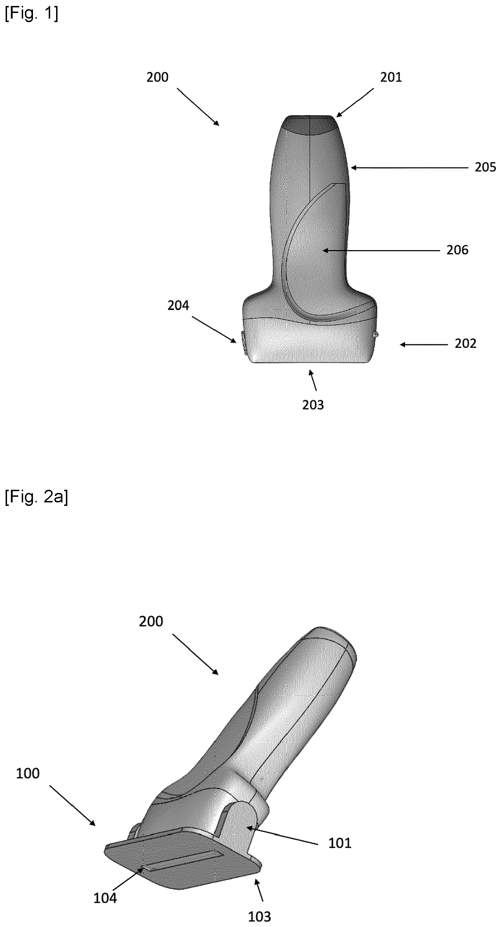

La figure 1 illustre schématiquement une sonde conventionnelle dans une première vue latérale. Ladite sonde 200 peut être utilisée (c'est-à-dire équipée ou associée) avec un dispositif selon la présente divulgation (non illustré sur la figure 1).

La sonde est configurée pour observer un milieu en acquérant des données du milieu, par exemple d'un corps humain ou animal. Par exemple, la sonde peut être une sonde à ultrasons. En d'autres termes, la sonde peut comprendre un ou plusieurs transducteurs ultrasonores. Néanmoins, la sonde peut comprendre aussi un autre type de capteur d'imagerie. La sonde peut donc aussi constituer une sonde d'imagerie dans n'importe quelle application, qui nécessite une inclinaison et/ou position stable de la sonde par rapport au milieu observé pendant l'acquisition des données. Selon un autre exemple la sonde peut comprendre un ou plusieurs dispositifs d'optique laser. Une telle sonde peut aussi être configurée pour une imagerie opto - acoustique utilisant des transducteurs ultrasonores et des dispositifs d'optique laser.

Généralement, la sonde peut être configurée pour une utilisation diagnostique et/ou thérapeutique. Par exemple, la sonde peut être configurée pour acquérir des données d'un milieu, par exemple pour imagerie du milieu, pour permettre un diagnostic. En outre, la sonde peut être configurée pour un procédé non invasif de focalisation d'ondes acoustiques dans un milieu, par exemple un milieu hétérogène dissipatif comprenant un milieu sensiblement homogène (par exemple, le cerveau) entouré au moins partiellement par une couche aberratrice dissipative (par exemple, le crâne). Les ondes acoustiques peuvent être émises depuis l'extérieur de la couche aberratrice et focalisées dans le milieu sensiblement homogène.

Par ailleurs, la sonde peut être configurée pour permettre la visualisation et le guidage de l'insertion d'une aiguille de biopsie en temps réel. Par exemple, la sonde peut collaborer avec un guide d'aiguille qui peut par exemple se fixer à la sonde pour le guidage de l'aiguille. Les données acquises par la sonde peuvent notamment être utilisées pour surveiller la biopsie. Selon un autre exemple, la navigation de l'aiguille peut superposer en temps réel l'échographie et des autres modes d'imagerie sur des volumes issus des modalités en coupe (IRM, CT, PET ou 3D-US).

La sonde peut aussi être utilisée pour surveiller et/ou guider l'implémentation des marqueurs de biopsie dans le milieu.

La sonde peut avoir des formes et/ou des réseaux de transducteurs différentes. Par exemple, la sonde peut être une sonde bidimensionnelle (par exemple avec un réseau linéaire de transducteurs), une sonde « 1.5D » (par exemple comprenant plusieurs types de transducteurs proposant ainsi des fonctionnalités différentes, par exemple un premier type de transducteurs en forme d'un réseau linéaire permettent une image échographique, et un second type de transducteurs destiné à la génération d'une contrainte mécanique interne permettant la propagation d'une onde de cisaillement dans le milieu, et disposé linéairement de part et/ou d'autre du premier type de transducteurs), une sonde « asymétrique » (par exemple avec un réseau avec un nombre d'éléments beaucoup plus important dans une dimension que dans l'autre), une sonde tridimensionnelle (par exemple avec un réseau avec le même nombre d'éléments dans les deux dimensions), une sonde courbe, une sonde matricielle, ou une sonde endocavitaire. La sonde 200 peut communiquer avec un système d'échographie et qui est configuré pour traiter des données d'un milieu acquises par la sonde. Elle peut par exemple faire partie d'un système d'échographie par ultrasons. Ce système peut par exemple être utilisé dans un contexte médical pour l'exam d'organes et/ou de tissus.

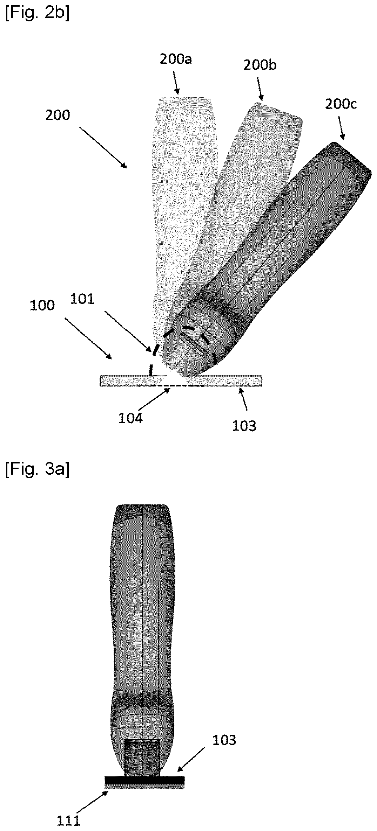

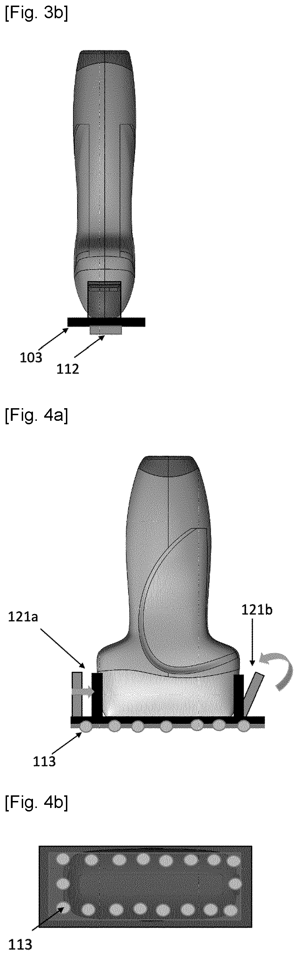

La sonde peut comprendre un premier côté plus large (illustré sur la figure 1, par exemple) et un deuxième côté plus étroit (illustré sur les figures 3a, 3b, par exemple).

La sonde inclut une enveloppe 205 qui est une coque renfermant les différents composants de la sonde. L'enveloppe 205 délimite un intérieur d'un extérieur de la sonde. L'enveloppe est par exemple globalement rigide pour faciliter la manipulation de la sonde. L'enveloppe 205 inclut selon un exemple une portion de préhension 206, globalement ergonomique, par laquelle un utilisateur manipule la sonde à une main. L'enveloppe 205 peut comprendre en outre une extrémité inférieure 201, où par exemple un câble ou une interface peut relier la sonde à une unité de traitement externe du système.

De plus, la sonde a une extrémité supérieure ou partie de tête 202. Cette extrémité supérieure peut être composée, d'une surface de transmission des ondes 203 qui est adaptée à être en contact avec la surface d'un milieu, par exemple la peau. La surface de transmission des ondes 203 est montrée dans les figures comme étant généralement plate, voire avec une légère courbure, planaire ou en 2D. surface de transmission des ondes 203 peut toutefois présenter diverses formes en 3D, avec possiblement une courbure prononcée.

L'enveloppe 205 est faite selon un exemple d'un ou plusieurs matériaux isolants électriquement comme par exemple du plastique, par exemple du type ABS. L'enveloppe peut être constituée de plusieurs parties assemblées. Par exemple l'enveloppe (sans la surface de transmission des ondes 203) pourrait être constituée par une coque d'une seule pièce de plastique laissant une ouverture adaptée pour y recevoir la surface d'émission et/ou réception, ainsi connectée et fermant l'enveloppe. L'enveloppe 205 peut être rigide ou flexible, en totalité ou en partie. Selon un exemple, la surface d'émission et/ou réception est constituée par un ou plusieurs polymère(s) flexible(s).

La sonde 200 inclut à l'intérieur de l'enveloppe un ou une pluralité des transducteurs, par exemple une pluralité d'éléments émetteurs et/ou récepteurs d'ondes acoustiques disposés à une première extrémité 202 de la sonde 200. Dans une variante, il est possible que la sonde 200 comprenne un seul élément émetteur et/ou récepteur. Selon un exemple, la pluralité d'éléments émetteurs et/ou récepteurs 120 comprend une pluralité d'éléments piézo-électriques. La pluralité des transducteurs ou d'éléments émetteurs et/ou récepteurs peut être disposée de façon à former une ligne ou front d'émission, ou bien une surface d'émission.

En outre, la sonde peut comprendre des moyens de fixation 204 sur ses seconds côtés plus étroits qui sont configurés pour fixer tout accessoire ou équipement.

La figure 2a représente schématiquement une vue en perspective d'un premier mode de réalisation d'un dispositif 100 (dans cet exemple accompagné de la sonde) selon la présente divulgation. Le dispositif 100 peut être configuré pour être associé à une sonde 200. Le dispositif est en outre configuré pour être maintenu contre ou pour être en contact avec une surface du milieu, lors de l'observation du milieu. Ainsi, le dispositif peut être disposé à l'extrémité supérieure 202 de la sonde. Plus particulièrement, le dispositif peut être disposé au niveau de la lentille 203 de la sonde, c'est-à-dire à l'endroit où les ondes ultrasonores peuvent être émises par la sonde.

Le dispositif est en particulier configuré pour être associé à la sonde 200 de manière pivotante, de sorte qu'un angle entre la sonde et la surface du milieu peut être ajusté en faisant pivoter la sonde par rapport au dispositif. Le dispositif peut rester pendant ce mouvement de pivotement dans la même position de contact sur la surface du milieu.

Le dispositif peut être configuré pour pouvoir pivoter autour d'au moins un axe par rapport à la sonde, en particulier autour d'un axe qui correspond à un axe d'une ligne de transducteurs de la sonde. Ledit axe peut s'étendre le long du premier côté plus large de la sonde, comme illustré par exemple sur la figure 2a. Toutefois, le dispositif peut également être configuré pour pouvoir pivoter autour de deux axes, qui s'étendent par exemple le long du premier côté large et de la direction des ondes, respectivement.

Le dispositif 100 peut être configuré pour être attaché et/ou fixé à la sonde 200. Par exemple, il peut comprendre deux éléments de maintien 101 qui sont configurés pour être fixés à la sonde en conservant le mouvement de rotation, notamment à ses moyens de fixation 204.

Le dispositif 100 peut en outre comprendre un plateau 103 configuré pour être disposé contre la surface du milieu et pour stabiliser la sonde avec un angle d'inclinaison choisi entre la sonde et la surface du milieu. En d'autres termes, le dispositif 100 peut avoir une surface plane qui est disposée sur la surface du milieu à étudier (c'est-à-dire être disposée sur la surface 203 de la sonde). Ledit plateau ou surface 103 doit être plus grand que la section transversale de la surface 203 de la sonde, afin de stabiliser une position inclinée de la sonde par rapport à la surface du milieu. En outre, le plateau 103 peut servir à fournir une pression homogène sur la surface du milieu. Ladite pression peut ainsi conduire à une déformation homogène du milieu. Ladite déformation homogène peut être avantageuse pour différents modes d'imagerie, par exemple pour l'élastographie non linéaire par ondes de cisaillement (en anglais « non-linear shear wave elastography » ; NL-SWE), comme décrit ci-dessus et dans WO2021116326A2.

Le dispositif 100 doit en outre comprendre une ouverture 104 avec des dimensions configurées pour permettre la transmission des ondes ultrasonores émises et/ou reçues par la sonde 200. Ladite ouverture 104 peut notamment être agencée dans le plateau 103, par exemple sous la forme d'un trou traversant. Les dimensions de l'ouverture peuvent être prédéfinies en fonction de la taille du réseau de transducteurs au sein de la sonde, notamment en fonction de la section transversale requise pour une transmission d'ondes non obstruée.

Le dispositif 100, ou au moins le plateau 103, peut être constitué de n'importe quel matériau rigide, par exemple un polymère ou un plastique. Le matériau du plateau peut être choisi de telle sorte qu'une déformation homogène du milieu puisse être obtenue, lorsque le plateau applique une pression sur la surface du milieu.

La figure 2b représente schématiquement une première vue en coupe du dispositif de la figure 2a, dans laquelle est illustré un mouvement de pivotement du dispositif par rapport à la sonde. Dans une position par défaut 200a, l'angle d'inclinaison peut être de 0°. Cela signifie que la sonde 200 peut être disposée perpendiculairement à la surface du dispositif 100 et donc à une surface de milieu sur laquelle le dispositif (c'est-à-dire le plateau 103) peut être placé.

Le dispositif 100 est configuré de telle sorte que la sonde peut être pivotée dans au moins une ou dans les deux directions de pivotement, comme illustré à la figure 2b, par exemple vers une position 200b et plus loin vers une position 200c. Le dispositif 100 peut ainsi être configuré pour permettre un angle d'inclinaison jusqu'à 20°, ou jusqu'à 30° ou même jusqu'à 45° (par exemple dans chacune des deux directions de pivotement).

Les moyens de fixation 101 ne sont indiqués que de manière schématique sur la figure 2b. Ces moyens de fixation peuvent avoir différentes configurations, comme décrit dans le contexte des autres modes de réalisation.

La figure 2b montre en outre de manière schématique l'ouverture 104 dans le plateau 103. Ladite ouverture 104 peut être formée de telle sorte qu'une transmission des ondes ultrasonores soit possible dans chaque angle d'inclinaison possible du dispositif. L'ouverture peut par exemple avoir la forme d'un "V" inversé (vu de la première vue en coupe, comme par exemple montré schématiquement sur la figure 2b). Ladite forme peut ainsi présenter une ouverture plus étroite vers la sonde et une ouverture plus large vers le milieu.

La figure 3a montre schématiquement une première vue latérale d'un deuxième mode de réalisation d'un dispositif selon la présente divulgation, dans lequel le dispositif peut avoir un film de couplage. Le deuxième mode de réalisation peut correspondre sensiblement au premier mode de réalisation.

En outre, le dispositif peut comprendre un moyen de couplage 111 sur une surface faisant face au milieu. Ladite partie peut être configurée pour supporter la transmission des ondes ultrasonores positionnées dans une zone où les ondes ultrasonores sont émises par la sonde. Grâce aux moyens de couplage, il peut ne pas être nécessaire d'appliquer un gel supplémentaire sur la surface du milieu, lorsque les données sont acquises. De par ses propriétés, le risque de glissement du dispositif sur la surface peut être réduit. De même, le risque de contamination infectieuse due à l'application d'un gel peut également être réduit.

Dans l'exemple de la figure 3a, le moyen de couplage peut consister en un coussinet de gel et/ou un film 111. Ce coussinet de gel et/ou ce film 111 peuvent couvrir toute la surface du plateau 103 ou seulement une partie de celle-ci. Par exemple, le coussinet de gel peut seulement couvrir ou remplir l'ouverture 104 (non représentée sur la figure 3a). Néanmoins, du gel peut être appliqué - sur la surface du plateau et/ou dans l'ouverture (par exemple dans le cas, où il n'y a pas de film).

Le moyen de couplage peut également comprendre un film bi-matériau 111 avec une partie adhérente positionnée dans une zone périphérique autour de l'ouverture. Le film bi-matériau 111 peut en outre comporter une partie de couplage (par exemple un film) dans une zone centrale couvrant l'ouverture.

La figure 3b montre schématiquement une première vue latérale d'un troisième mode de réalisation d'un dispositif selon la présente divulgation, dans lequel le dispositif peut avoir du gel. Le troisième mode de réalisation peut correspondre sensiblement au premier ou au deuxième mode de réalisation.

En outre, le dispositif peut comprendre un moyen de couplage 112 sur une surface faisant face au milieu. Dans l'exemple de la figure 3b, le moyen de couplage peut consister en une pellicule de gel 112 couvrant ou remplissant l'ouverture 104. Le coussinet de gel peut cependant aussi recouvrir la section transversale de l'ouverture sur la surface du dispositif qui fait face au milieu. Il est à noter que la figure 3b n'illustre que schématiquement le coussinet de gel. Le coussinet de gel peut notamment être plan avec la surface du plateau 103 qui fait face au milieu, c'est-à-dire qu'il ne fait pas saillie de cette surface.

La figure 4a montre schématiquement une deuxième vue latérale d'un quatrième mode de réalisation d'un dispositif selon la présente divulgation, dans lequel le dispositif peut avoir un moyen de stabilisation configuré pour optimiser la stabilité du dispositif sur la surface du milieu, par exemple sous la forme d'une partie adhérente avec des moyens anti-glissement pour réduire le glissement du dispositif et des points d'attache adaptables. La figure 4b représente schématiquement une vue de dessous du dispositif de la figure 4a et notamment la disposition des points anti-glissement. Ladite vue de dessous peut en particulier montrer la surface du dispositif qui est configurée pour faire face au milieu. Le quatrième mode de réalisation peut correspondre sensiblement à l'un des quelconques modes de réalisation susmentionnés.

Le dispositif peut en outre comprendre un moyen de stabilisation configuré pour optimiser la stabilité du dispositif sur la surface du milieu, par exemple sous la forme de parties adhérentes 113 sur une surface du dispositif configurée pour être disposée contre la surface du milieu. Dans l'exemple des figures 4a et 4b, les parties adhérentes 113 peut avoir la forme de points anti-glissement 113. Les points 113 peuvent par exemple être constitués d'un matériau en caoutchouc. Comme illustré sur la figure 4b, les parties adhérentes peuvent notamment être disposées dans une zone périphérique autour de l'ouverture. De cette manière, les parties adhérentes peuvent empêcher de manière fiable le glissement du dispositif sur la surface du milieu. En même temps, elles ne perturbent pas la transmission des ondes ultrasonores à travers l'ouverture.

Le dispositif peut en outre comprendre des bras de maintien 121a, 121b configurés pour être fixés à la sonde, par exemple à ses moyens de fixation 204. Les bras de maintien 121a, 121b peuvent faire saillie d'une surface arrière du plateau 103 qui est opposée à la surface qui fait face au milieu.

Les bras de maintien peuvent par exemple comprendre des tiges faisant saillie des bras vers les moyens de fixation 204 de la sonde. Les tiges peuvent être formées de manière à pouvoir être reçues et fixées de manière pivotante aux moyens de fixation 204.

Le dispositif peut être configuré pour une attache et/ou un détachement rapide à/de la sonde. Dans l'exemple illustré, au moins le bras de maintien 121a peut être configuré pour être déplacé transversalement sur la surface arrière, afin de permettre une fixation du dispositif à des coques de sondes de différentes tailles. Selon un autre exemple, le dispositif peut comprendre un système d'étau. Dans cet exemple, au moins le bras de maintien 121b peut être configuré pour être déplacé de manière pivotante sur la surface arrière du plateau, de sorte que le bras de maintien puisse être clipsé sur et déclipsé de la sonde. En conséquence, le dispositif peut être configuré pour une fixation et une libération rapide. Cependant, les deux bras de maintien peuvent également avoir la fonction de déplacement et/ou de pivotement décrite ci-dessus.

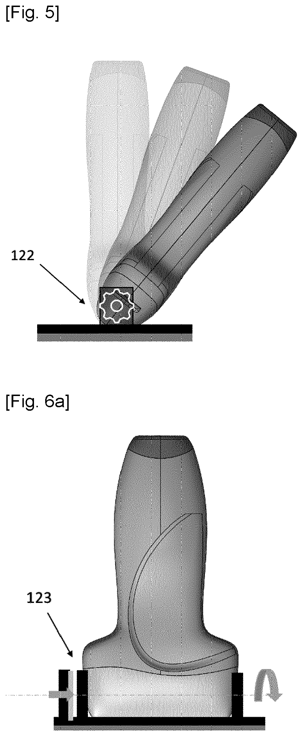

La figure 5 montre schématiquement une première vue latérale d'un cinquième mode de réalisation d'un dispositif selon la présente divulgation, dans lequel le dispositif peut avoir un moyen mécanique pour un pivotement par étapes et/ou d'indexation. Le cinquième mode de réalisation peut correspondre sensiblement à l'un des quelconques modes de réalisation susmentionnés.

Comme illustré schématiquement, au moins un des bras de maintien peut comprendre un mécanisme d'engrenage 122 (ou tout autre mécanisme) qui est configuré pour permettre uniquement un pivotement par étapes / paliers et/ou d'indexage (c'est à dire par crans). En d'autres termes, le mécanisme 122 peut permettre une fixation du dispositif à la sonde uniquement à des angles d'inclinaison prédéfinis.

La figure 6a montre schématiquement une deuxième vue latérale d'un sixième mode de réalisation d'un dispositif selon la présente divulgation, dans lequel le dispositif peut permettre un pivotement continu. Selon une option, le dispositif peut comprendre un moyen électromécanique (ou d'autre moyen mécanique) configuré pour le maintien en position et/ou l'ajustement de l'angle du dispositif. Le sixième mode de réalisation peut correspondre sensiblement à l'un des modes de réalisation susmentionnés.

En outre, au moins un des bras de maintien peut comprendre un mécanisme d'embrayage 123 (ou tout autre mécanisme) qui est configuré pour permettre un pivotement continu du dispositif par rapport à la sonde. De plus, le mécanisme 123 peut permettre un maintien du dispositif à tout angle d'inclinaison sélectionnable, par exemple en actionnant l'embrayage. En conséquence, le mécanisme d'embrayage peut constituer une alternative au mécanisme d'engrenage 122 de la figure 5.

Le mécanisme d'embrayage peut par exemple comprendre un premier disque d'embrayage qui est configuré pour être fixé à la sonde, de sorte qu'une rotation du premier disque d'embrayage par rapport à la sonde peut être empêchée. Un second disque d'embrayage peut être fixé au plateau 103, empêchant toute rotation du second disque d'embrayage par rapport au plateau mais permettant un mouvement transversal du second disque d'embrayage par rapport au plateau. Ce mouvement transversal permet d'actionner l'embrayage.

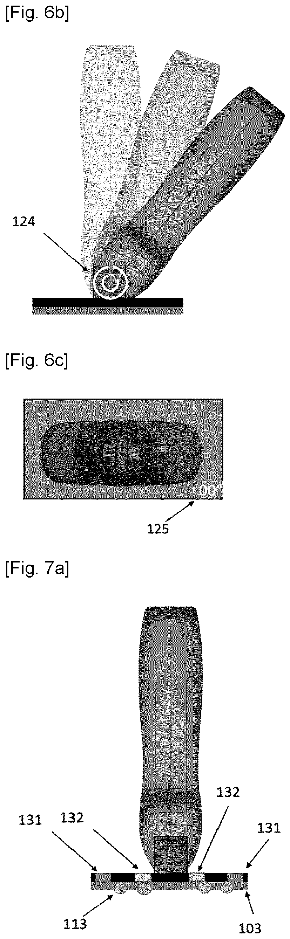

La figure 6b représente schématiquement une première vue latérale du dispositif de la figure 6a avec un capteur d'inclinaison optionnel 124. Le capteur d'inclinaison peut par exemple être intégré au mécanisme d'embrayage 123. Le capteur d'inclinaison peut être configuré pour mesurer un angle d'inclinaison du dispositif par rapport à la sonde. Le capteur d'inclinaison peut être un capteur électronique.

La figure 6c représente schématiquement une vue de dessus du dispositif des figures 6a et 6b avec un indicateur d'inclinaison 125 optionnel. L'indicateur d'inclinaison peut par exemple être un afficheur électronique. L'indicateur peut être configuré pour afficher l'angle d'inclinaison, tel que mesuré par le capteur d'inclinaison 124.

La figure 7a représente schématiquement une première vue latérale d'un septième mode de réalisation d'un dispositif selon la présente divulgation, dans lequel le dispositif peut comporter des capteurs de pression 131 et/ou des capteurs de température 132. Le septième mode de réalisation peut correspondre sensiblement à l'un quelconque des modes de réalisation susmentionnés.

Ainsi, le dispositif peut comprendre au moins un capteur de pression qui est configuré pour mesurer une pression appliquée via le dispositif (notamment son plateau 102) sur la surface du milieu. Cette valeur de pression peut être utilisée par exemple comme guide dans une méthode NL-SWE (ou plus généralement pour déterminer un niveau de déformation du milieu).

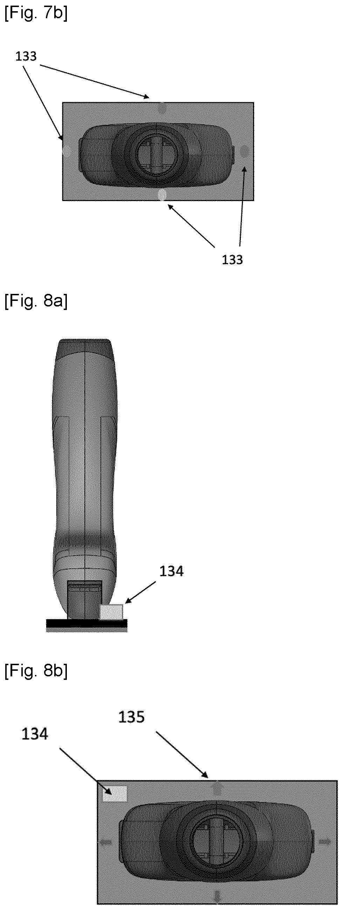

Cependant, comme le montre la figure 7a, le dispositif peut également comprendre une pluralité de capteurs de pression 131. Lesdits capteurs 131 peuvent être disposés dans différentes zones du plateau 103, par exemple autour de l'ouverture 104. Ainsi, lesdits capteurs peuvent être configurés pour mesurer une pression locale appliquée par la zone respective du plateau 103 à la surface du milieu. Dans un exemple, le dispositif peut comprendre quatre capteurs de pression 131 (ou tout autre nombre) configurés pour mesurer la pression locale sur les quatre côtés du plateau 103 (par exemple de forme rectangulaire). En conséquence, les capteurs de pression 131 peuvent être situés de manière à correspondre aux emplacements des indicateurs de pression 133, comme illustré sur la figure 7b.

Le dispositif peut également comprendre un ou plusieurs capteurs de température 132. Ces capteurs peuvent être configurés pour mesurer la température de la surface du milieu ou de la surface de la sonde. La température mesurée peut être utilisée par exemple pour surveiller un éventuel échauffement du milieu dû aux ondes ultrasonores émises par la sonde. Par exemple, dans le cas où la température dépasse une valeur prédéfinie, une information ou une alarme peut être signalée à l'utilisateur de la sonde.

Le dispositif peut également comprendre des parties adhérentes sous la forme de points antidérapants 113, comme décrit dans le contexte des figures 4a et 4b.

La figure 7b représente schématiquement une vue de dessus du dispositif de la figure 7a. La figure 7b montre en particulier la disposition des indicateurs de pression.

Le dispositif peut comprendre au moins un indicateur de pression 133 qui est configuré pour indiquer une pression appliquée avec le dispositif, notamment son plateau 102, sur la surface du milieu. Ladite valeur de pression peut être déterminée par exemple par le capteur de pression 132. L'indicateur de pression peut comprendre ou être constitué d'un élément lumineux, par exemple une LED. En changeant sa couleur de lumière, l'élément d'illumination peut ainsi indiquer un niveau de pression.

L'indicateur de pression peut être disposé sur la surface arrière du plateau 103.

Dans un autre exemple, la couleur de la lumière peut indiquer un niveau de pression qualitatif. Ce niveau de pression qualitatif peut être déterminé par exemple par une comparaison entre une pression réelle (par exemple mesurée par le capteur de pression) et une pression cible. Cette pression cible peut être déterminée dans le cadre d'une méthode NL-SWE, par exemple. Dans cette méthode, différents niveaux de déformation du milieu peuvent être requis pendant l'acquisition des données par la sonde. Sur la base d'un niveau de déformation requis, une pression cible respective peut être déterminée. Dans un exemple, les couleurs de lumière qualitative peuvent comprendre : « vert » (représentant une pression acceptable), « rouge » (représentant une pression trop forte), et toute autre couleur comme le jaune ou le bleu (représentant une pression trop faible).

Cependant, comme le montre la figure 7b, le dispositif peut également comprendre une pluralité d'indicateurs de pression 133. Ces indicateurs 133 peuvent être disposés dans différentes zones du plateau 103, par exemple autour de la zone couverte par la section transversale de la sonde vue de dessus (cf. la vue de la figure 7b). Lesdits indicateurs peuvent être configurés pour indiquer une pression locale telle que mesurée par des capteurs de pression 131 respectivement disposés. Dans un exemple, le dispositif peut comprendre quatre indicateurs de pression 133 disposés et configurés pour indiquer la pression locale sur les quatre côtés du plateau 103 (par exemple de forme rectangulaire).

La figure 8a représente schématiquement une première vue latérale d'un huitième mode de réalisation d'un dispositif selon la présente divulgation, dans lequel le dispositif peut comporter un capteur de positionnement et des indicateurs de direction. Le huitième mode de réalisation peut correspondre sensiblement à l'un des quelconques modes de réalisation susmentionnés.

Le dispositif peut comprendre un capteur de positionnement 134, par exemple sous la forme d'une étiquette RFID, des capteurs d'un champ magnétique, et/ou d'une ou plusieurs caméras. Le capteur de positionnement peut par exemple transmettre les données acquises au système, avec lequel la sonde communique. Le système peut alors mettre en oeuvre une méthode de localisation, comme décrit ci-après.

L'étiquette RFID peut être configurée pour émettre un signal grâce auquel le dispositif peut être localisé. Lesdites informations de localisation peuvent être utilisées conjointement avec des informations externes d'une région cible sur la surface du support, afin de déterminer une direction de déplacement requise du dispositif. La région cible peut être une région où les données doivent être acquises par la sonde. Une région cible peut être identifiée par un algorithme mis en œuvre par ordinateur et exécuté par le système, par exemple un algorithme basé sur l'IA (intelligence artificielle).

La caméra peut être configurée pour balayer la surface du support. Sur la base des données de la caméra, une position actuelle du dispositif par rapport à la surface du milieu peut être déterminée. Lesdites informations de localisation peuvent être utilisées conjointement avec des informations externes d'une région cible sur la surface du milieu, afin de déterminer une direction de mouvement requise du dispositif.

Il est également possible d'utiliser les données acquises par la sonde pour déterminer la position actuelle du dispositif par rapport à la surface du support. Dans ce cas, le capteur de positionnement 134 peut ne pas être nécessaire.

La figure 8b représente schématiquement une vue de dessus du dispositif de la figure 8a. En particulier, la figure 8b montre des indicateurs de direction 135.

Le dispositif peut comprendre au moins un indicateur de direction 135 qui est configuré pour indiquer une direction vers une région cible. L'indicateur de direction peut comprendre ou consister en un affichage ou un élément d'éclairage, par exemple une LED. En changeant sa couleur de lumière, l'élément d'éclairage peut indiquer si une région cible a été atteinte.

L'indicateur de direction peut être disposé sur la surface arrière du plateau 103.

Toutefois, comme illustré à la figure 8b, le dispositif peut également comprendre une pluralité d'indicateurs de direction 133. Ces indicateurs 135 peuvent être disposés dans différentes zones du plateau 103, par exemple autour de la zone couverte par la section transversale de la sonde vue de dessus (cf. la vue de la figure 7b). Lesdits indicateurs peuvent être des éléments d'éclairage. Dans un exemple, le dispositif peut comprendre quatre indicateurs de direction 135 sous la forme de flèches lumineuses. Les quatre indicateurs de direction 135 peuvent être disposés sur les quatre côtés du plateau 103 (par exemple de forme rectangulaire). En allumant la ou les flèches respectives, un utilisateur de la sonde peut être guidé vers la région cible.

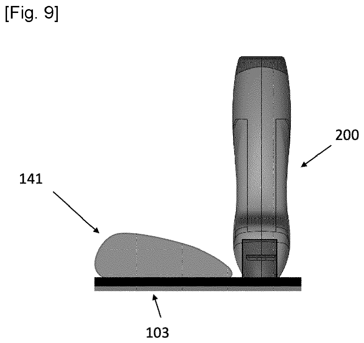

La figure 9 représente schématiquement une première vue latérale d'un neuvième mode de réalisation d'un dispositif selon la présente divulgation, dans lequel le dispositif peut comporter un support ergonomique 141, par exemple un coussin et/ou poignée ergonomique. Le support ergonomique peut être configuré de telle sorte qu'une main d'un utilisateur peut tenir la sonde et en même temps reposer sur le coussin. En conséquence, l'utilisation de la sonde est plus confortable pour un utilisateur, lorsque le dispositif est fixé. En même temps, la main de l'utilisateur peut appliquer une pression via le support ergonomique sur la surface du support.

Le support ergonomique 141 peut être positionné sur la surface arrière du plateau 103. Le plateau peut être fait de n'importe quel matériau rigide (comme le plastique) et peut donc conduire à une pression homogène, même si le support ergonomique est souple.

En outre, le plateau peut être agrandi pour offrir une plus grande surface de la face arrière sur au moins un côté de la sonde. En conséquence, le support ergonomique peut être placé sur ladite surface élargie et peut donc également avoir une taille plus grande et plus confortable.

Tous ces modes de réalisations et d'autres exemples tels que décrits ci-dessus sont donnés uniquement à titre d'exemple non limitatif, et peuvent être combinés et/ou modifiés selon la portée des revendications suivantes.

## Revendications

1. Dispositif pour une sonde d'échographie pour acquérir des données d'échographie d'un milieu, dans lequel
le dispositif est configuré pour être associé à la sonde d'une manière pivotante, tel que l'angle entre la sonde et une surface du milieu peut être ajusté et/ou fixé en pivotant le dispositif.

2. Le dispositif selon la revendication 1 ou 2, comprenant en outre :
des moyens adaptables configurés pour attacher le dispositif à des coques de sondes différentes.

3. Le dispositif selon l'une quelconque des revendications précédentes, dans lequel
la sonde est configurée pour émettre des ondes ultrasonores et/ou comprend un transducteur configuré pour émettre des ondes ultrasonores sur un premier côté de la sonde, et le dispositif est configuré pour être positionné et/ou attaché sur le premier côté de la sonde d'une manière pivotante.

4. Le dispositif selon l'une quelconque des revendications précédentes, comprenant en outre :
une ouverture configurée pour permettre la transmission des ondes ultrasonores.

5. Le dispositif selon l'une quelconque des revendications précédentes, comprenant en outre :
un plateau et/ou une surface plane configuré(e) pour être disposé(e) contre la surface du milieu et pour stabiliser l'angle entre la sonde et la surface du milieu.

6. Le dispositif selon l'une quelconque des revendications précédentes, dans lequel
le dispositif et/ou son ouverture comprend un moyen de couplage configuré pour permettre la transmission des ondes ultrasonores.

7. Le dispositif selon la revendication précédente, dans lequel
le moyen de couplage comprend un film et/ou un coussinet de gel, et optionnellement un adhésif pour attacher le film et/ou le coussinet de gel au dispositif.

8. Le dispositif selon l'une quelconque des revendications précédentes 8 ou 9, dans lequel
le moyen de couplage comprend un film bi-matière avec une ou plusieurs parties adhérentes positionnées dans une zone périphérique et une partie configurée pour coupler le dispositif au milieu et/ou pour permettre la transmission des ondes ultrasonores et positionnée dans une zone où des ondes ultrasonores sont émises par la sonde.

9. Le dispositif selon l'une quelconque des revendications précédentes, dans lequel
le dispositif et/ou son ouverture comprend un moyen de stabilisation configuré pour optimiser la stabilité du dispositif sur la surface du milieu.

10. Le dispositif selon la revendication précédente, dans lequel
le moyen de stabilisation comprend une partie adhérente sur une surface du dispositif configurée pour être disposée contre la surface du milieu.

11. Le dispositif selon l'une quelconque des revendications précédentes, dans lequel
le dispositif est configuré pour ajuster et/ou fixer l'angle en utilisant des moyens mécaniques et/ou électromécaniques et/ou électroniques,
et/ou
des moyens mécaniques comprennent un embrayage pour le réglage de l'angle et/ou pour fixer l'angle, et/ou
le dispositif est configuré pour un réglage de l'angle par étapes ou en continu.

12. Le dispositif selon l'une quelconque des revendications précédentes, comprenant en outre au moins un des éléments suivants :
un capteur d'inclinaison configuré pour mesurer l'angle entre la sonde et la surface du milieu,
un afficheur pour indiquer l'angle,
un capteur de température configuré pour mesurer la température de la surface du milieu et/ou à la surface de la sonde,
un capteur de pression configuré pour mesurer une pression appliquée à la surface du milieu par le dispositif,
un indicateur de pression configuré pour indiquer un niveau de pression appliquée à la surface du milieu, et/ou
une pluralité de capteurs de pression locale, chacun étant configuré pour mesurer une pression appliquée à la surface du milieu par une zone différente du dispositif,
et optionnellement une pluralité d'indicateurs de pression locale, dont chaque indicateur de pression est configuré pour indiquer un niveau de pression locale.

13. Le dispositif selon la revendication précédente, dans lequel l'indicateur de pression et/ou les indicateurs de pression locale sont disposés sur un côté opposé d'une surface du dispositif qui est configurée pour être disposée contre le milieu.

14. Le dispositif selon l'une quelconque des revendications précédentes, comprenant en outre:
un système de guidage de positionnement configuré pour indiquer une position cible de la sonde à la surface du milieu, en guidant la sonde ou un utilisateur de la sonde vers la position cible.

15. Le dispositif selon la revendication précédente, dans lequel le système de guidage de positionnement comprend :
des indicateurs de direction situés à la surface du dispositif et configurés pour indiquer la direction vers la position cible, et/ou
des moyens de localisation configurés pour localiser la position du dispositif par rapport au milieu.

16. Le dispositif selon l'une quelconque des revendications précédentes 14 ou 15, dans lequel
le système de guidage de positionnement est configuré pour communiquer avec une unité de traitement externe pour transmettre des données de localisation et/ou pour recevoir des données d'une position cible et/ou d'une direction vers la position cible.

17. Le dispositif selon l'une quelconque des revendications précédentes, comprenant en outre :
un support ergonomique configuré tel qu'une main tenant la sonde puisse reposer dessus.

18. Sonde d'échographie pour acquérir des données d'échographie d'un milieu, configurée pour collaborer avec un système destiné à traiter les données d'échographie, la sonde comprenant un dispositif selon l'une des quelconques revendications précédentes.

19. Système d'échographie, comprenant :
un dispositif selon l'une des quelconques revendications précédentes 1 à 17, optionnellement une sonde d'échographie pour acquérir des données d'un milieu, et des moyens de localisation configurés pour localiser la position actuelle du dispositif par rapport au milieu.

20. Procédé pour observer un milieu par une sonde d'imagerie en utilisant un dispositif associé à la sonde d'une manière pivotante, comprenant les étapes :
A. ajuster et/ou fixer l'angle entre la sonde et la surface du milieu en pivotant le dispositif,
B. acquérir des données du milieu en utilisant la sonde.

21. Le procédé selon la revendication précédente, comprenant en outre les étapes :
C1. changer un niveau de déformation du milieu appliqué par le dispositif, et/ou
C2. changer l'angle.

22. Le procédé selon l'une quelconque des revendications précédentes 20 ou 21, comprenant au moins une des réitérations suivantes :
réitérer les étapes A, B,
réitérer l'étape B après l'étape C1 et/ou C2,
réitérer les étapes B, et au moins un de C1, C2, et
réitérer les étapes A, B, et au moins un de C1, C2.
